Europäisches Patentamt

European Patent Office

Office européen des brevets

(19)

(11) Publication number: **0 016 268
B1**

(12) **EUROPEAN PATENT SPECIFICATION**

(45) Date of publication of patent specification: **08.06.83**

(51) Int. Cl.³: **A 61 F 1/04**

(21) Application number: **79300423.5**

(22) Date of filing: **16.03.79**

(54) **Lockable hinge joint for limb supports and other orthopaedic appliances.**

(43) Date of publication of application:
**01.10.80 Bulletin 80/20**

(45) Publication of the grant of the patent:
**08.06.83 Bulletin 83/23**

(84) Designated Contracting States:
**BE DE FR IT LU NL**

(56) References cited:
**DE - B - 1 193 636
DE - B - 2 119 599
DE - C - 298 767
US - A - 2 591 373**

(73) Proprietor: **ROBERT KELLIE & SON LIMITED
Rutherford Road Dryburgh Industrial Estate
Dundee DD2 3XF Scotland (GB)**

(72) Inventor: **Fyffe, James
75 East Haddon, Craighiebank
Dundee Scotland (GB)**
Inventor: **Duthie, James Eric
29 Melfort Place
Dundee Scotland (GB)**

(74) Representative: **Cole, Paul Gilbert et al,
NPM Hughes Clark Andrews & Byrne 5 Stone
Buildings Lincoln's Inn
London WC2A 3XT (GB)**

## Lockable hinge joint for limb supports and other orthopaedic appliances

The present invention relates to a lockable joint which is particularly, but not exclusively, useful as a knee joint of an orthopaedic leg support.

A leg brace includes thigh supports ("steels") to either side of the thigh and calf steels to either side of the lower part of the leg, the thigh and shank steels on each side of the leg being connected by a pivot joint at knee level. The pivot joint on one or both sides of the knee is arranged to be locked when the patient is standing or walking but has to be releaseable to allow the knee joint to flex when the patient is sitting. The release mechanism may be operated by a ring catch or drop lock which is a sliding ring on the thigh steel which may be lowered into engagement with the pivot joint to lock it and which is lifted to release the joint. Another kind of lock known as a bar lock or French lock involves a hinged release member pivoted to the thigh steel and operated by lifting an extension bar attached to the release member. Where bar locks are used, the pivot joints on both sides of the knee are normally lockable and the release members on opposite sides of the knee are interconnected by means of a bar extending around the back of the knee. A further kind of release mechanism employs a hinged release member having an extension bar to which a lifting cable may be fixed. The patient's disability may often affect parts of his body other than his legs, and the orthopaedic fitter has to select from the range of available release mechanisms one which is within the patient's capacity to operate.

Known types of release mechanisms and in particular the ring catch and bar lock mechanisms operate on different principles and require differently shaped hinge members. The fact that different kinds of release mechanism require the differently shaped hinge members and different ancillary parts decreases the length of production runs and increases the number of parts required to be stocked by a limb fitting centre, both of which are factors tending to increase the cost of orthopaedic limb supports. It is an object of the invention to provide a release mechanism in which the same hinge members may be used in association with different release mechanisms and in particular in association with a release operating analogously to a ring catch by upward finger pressure from the rear of the release mechanism and also in association with a bar lock, or cable release, the only modification required being to the release member.

Manually operated ring catches are not normally spring loaded into the locked position and suffer from the disadvantage that they may accidentally be shaken out of engagement while the patient is walking. However, the joint of the invention may be released by upward finger pressure like a manual ring catch but the release member is spring loaded into the locked position. A further disadvantage of currently available ring catches is that although they may have little play when new, significant play is liable to develop as a result of wear, but such a tendency is not significantly exhibited by the hinge joint of the invention.

U.S. Patent No. 2591373 describes a lockable knee joint for an artificial limb in which the lower end of an upper brace member is secured to a sleeve provided with circular extensions that project downwardly in lapped relationship on each side of the upper end of a lower brace member, the two being connected by a hinge pin for relative flexing movement. The upper end of the lower brace member is provided with an anteriorly disposed segmental arcuate bearing surface concentric with the hinge pin and a posterior arcuate recess inwards of the arcuate bearing surface. The upper brace member is provided with a locking lug that projects downwardly into the arcuate recess and in the unflexed position of the joint abuts the posterior end of the arcuate bearing surface to prevent movement beyond the unflexed position. The arcuate bearing surface terminates at its anterior end in an anterior recess which aligns with a slot in the upper member when the joint is unflexed and extends axially away from said recess. A detent is resiliently biased by a spring into a position where it engages the anterior recess to prevent unintentional joint flexion and is retractible into the slot by pulling on a knob that raises a narrow metal lock operating finger connected to detent through a pin so that the detent, pin and operating finger slide up and down together. The detent is supported or supportable on the arcuate bearing surface when the joint is flexed and is urged into engagement with the recess when the joint is straightened to lock the joint in the unflexed position. But the detent is not lever-actuated and is unsuitable for lever actuation because both the detent and the slot are located at the front edge of the joint.

Broadly stated the invention provides a releasably lockable joint for an orthopaedic limb support or for an artificial limb, which joint comprises first and second members pivoted together at their ends for relative flexing movement and having stop faces which abut when the joint is straight to prevent movement beyond the straight position, the pivoted end of the first member having an arcuate portion which terminates in a recess or shoulder and the second member having a channel which is in register with the base of said recess or shoulder when the joint is straight and which extends away from the recess or shoulder, a detent slidable in the channel so that it may be retracted into the channel by operation of a release and is supported or supportable in its retracted position by said arcuate portion when the joint is flexed but is urged by reslient means into engagement with said recess or shoulder when the joint is straightened to lock the joint in the straight position, characterised in that the release for the detent is a lever pivoted at one end to one longitudinal edge of the second member and extending transversely of the second member so that its other end projects beyond the other longitudinal edge of the second member with a pivotal connection to the detent at an intermediate position along the length of the release lever.

The lockable joint may additionally comprise a releaseable catch for locking the detent in the retracted position. Such a catch may comprise an auxiliary channel in the second member opening into the detent channel, a ball catch in the auxiliary channel resiliently biased to project therefrom and a recess in the detent positioned to be engaged by the ball of the ball catch when the detent is retracted. The detent is preferably wedge shaped, with its rear face which engages an upwardly directed locking face of the recess or shoulder in the first member inclined at a small acute angle typically of the order of 10 degrees with respect to the other face thereof, the locking face of the recess or shoulder being directed parallel to the inclined face of the detent when the joint is straight so that when the detent is slid into engagement with the shoulder or recess, its inclined face is wedged against the lock face which wedging action minimises play in the pivot. A further factor tending to minimise play is that the interengaging faces of the detent and the shoulder or recess may be directed at approximately a right angle to the stop faces when the joint is in the straight position and the interengaging faces and the stop faces are a quarter circle removed from one another with respect to the pivot axis and so exert a wedging action taking up any play in the pivot.

The release lever is preferably in the form of an external ring fitted to the second member with its front edge pivotally received in a recess in the front edge of the second member and the internal surface of its rear edge normally spaced from the rear edge of the second member, an upward movement of the rear edge of the ring retracting the detent into the channel. The rear portion of the ring may be further provided with a socket for receiving an extension lever which forms part of a bar lock or cable lift.

Preferably the joint is manufactured as a sub-assembly separate from the steels of the limb support into which it is to be incorporated. The outer ends of the first and second members are preferably formed with a internal axial channel dimensioned to receive the end of the respective steel which is then screwed or riveted in place. The end of the member may have its sides split longitudinally so that fixing screws can be passed through each side of the member and through the steel to force the sides together and clamp the steel rigidly therebetween.

Various embodiments of the invention will now be described by way of example with reference to the accompanying drawings in which similar parts are denoted by similar numerals and:

Figure 1 is a side view of a manually lockable joint for an orthopaedic limb support with the second member shown partly in section, the joint being in the straight position;

Figure 2 is a side view of the joint shown in Figure 1 in a flexed position;

Figure 3 is a side view of a joint similar to that shown in Figures 1 and 2 but arranged for automatic locking;

Figures 4(I) and 4(III) are side views of two different forms of pivoting ring catch fitted to the second member;

Figures 4(II) and 4(IIa) are side views of the first and second members arranged to be operated by a bar lock and in the straight and in a flexed position respectively; and

Figure 4(IV) is a side view of a second member arrange to be operated by means of a cable lift.

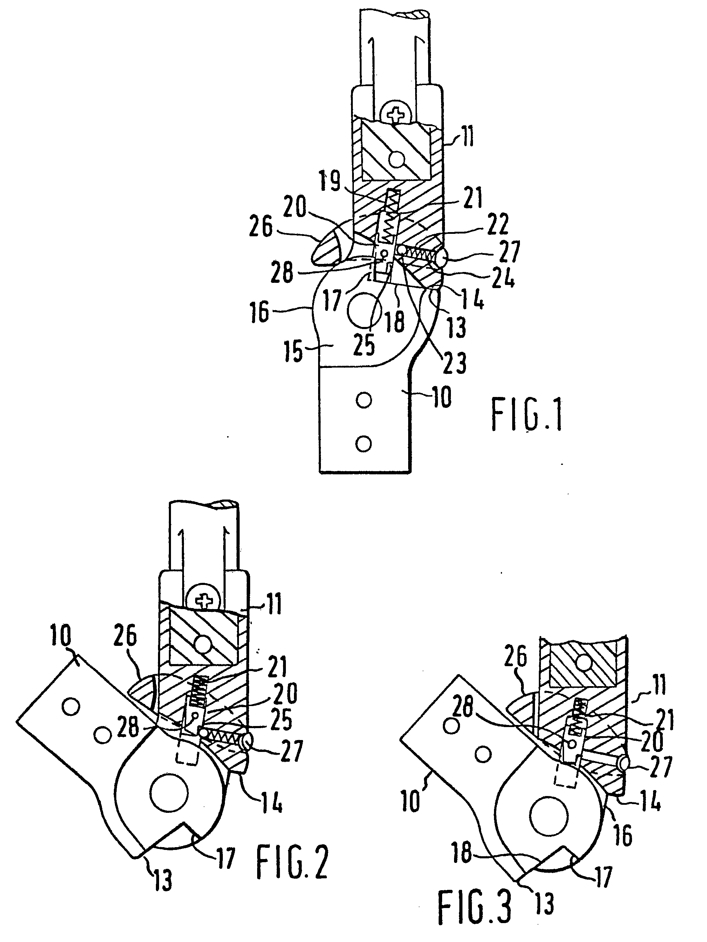

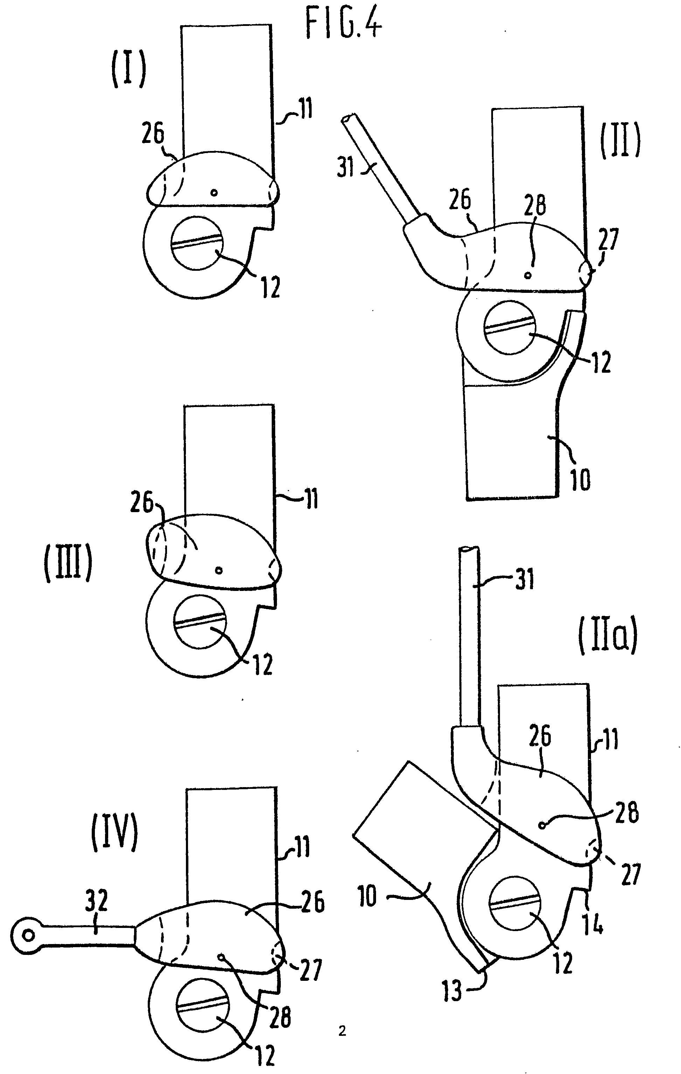

In Figure 1, a knee joint for attachment to side steels of an orthopaedic leg brace comprises a first or calf member 10 having a head in the form of an eye-bolt pivoted to a second or thigh member 11 by means of a pivot bush (not shown) which passes through the eyes of the bolt heads and is held in place by means of a locking screw 12 (Figure 4). The head of the member 11 is split to form a fork bracket having two transversely spaced apart circular leaves which, as is apparent from Figure 4(IIa) are angled rearwardly so that the centre of the locking screw 12 is offset a small distance rearwardly of the longitudinal centre line of the thigh member 11. The forward end of the member 11 extends beyond the base of the two-leaf fork brackets to define a transversely directed limit face 14. The eye-bolt portion of the member 10 is formed with a zone 15 of reduced thickness which fits between the leaves of the fork bracket and has at its front edge a full thickness region outside the periphery of the leaves and bounded at its upper end by a transversely directed stop face 13. The stop faces 13 and 14 abut when the joint is unflexed to prevent further relative angular movement of the members 10 and 11. The stop face 13 is formed on a portion of the member 10 which is of full thickness so that there is an ample area of contact with the stop face 14, and the faces 13 and 14 are well spaced from the pivot axis of the joint so that the unit load on the faces 13 and 14 in the unflexed joint is low and the rate of wear is minimised. It will be noted that in the unflexed joint the axis of the member 11 is generally parallel to and offset forwardly of the axis of the member 10.

The eye-bolt portion of the member 10 is formed with an outer peripheral edge 16 of the zone of reduced thickness which terminates in a shoulder defined by a lock face 17 directed inwardly parallel to the longitudinal direction of the member 10 and a transverse face 18 which is continuous with the stop face 13. A rectangular slot or channel 19 in the member 11 is directed at a small acute angle, typically about 10 degrees, to the axis of the member 11 with its lower end positioned so that its rear transverse face registers with the lock face 17 on member 10 when the joint is unflexed. A locking plunger or detent 20 slidable in the channel 19 is urged into locking engagement with the lock face 17 by means of a spring 21. It will be noted that the rear face of the detent is directed at a small acute angle (typically about 10 degrees) with respect to the longitudinal direction of the channel 19 and is parallel to the lock face 17 on the member 10 when the joint is in the straight position. The detent 20 is dimensioned to engage the lock face 17 with its end clear of the bottom face 18 and exerts a wedging action on the lock face 17 when the joint is unflexed so that there is minimal play. In a typical joint the detent and lock face engage over a distance of 3 mm or more and when the joint is new there is a distance of 1.5 mm or more between the end face of the detent and the bottom face 18 of the shoulder for wear take-up. The joint has the advantage that wear of the detent and lock face does not give rise to play but instead the wear is automatically taken up by engagement of the wedge with the lock face 17 at a detent position closer to the bottom face 18 of the shoulder than it occupied when the joint was new.

Figures 1 and 2 illustrate a joint which is arranged to be held unlocked until deliberately set to lock. An auxiliary bore 22 extends into the channel 19 from the front face of member 11 and has a catch formed by a ball 23 and spring 24 which as shown in Figure 2 urges the ball 23 into engagement with a rebate 25 in the front face of the detent 20 to hold the detent in a retracted position.

An actuating ring 26 is fitted over the member 11 with its front portion pivotally received in a recess 27 in the front edge of the member 11 and is connected at an intermediate position to the detent 20 by means of a pivot pin 28. In the locked position shown in Figure 1 the actuating ring is urged downwardly by means of the detent spring 21 but its rear face may be lifted as shown in Figure 2 to retract the detent 20 into the channel 19 and compress the spring 21.

In use as a knee joint of an orthopaedic leg brace, the joint is locked when the patient is standing or walking. When he or she wishes to sit down, the rear end of the actuating ring is lifted to retract the detent out of engagement with the lock face 17 and into the channel 19 where it is held in a retracted position by engagement of the ball catch 23, 24 with the rebate 25. When the patient stands up and the joint is straightened, the rear end of the actuating ring is depressed to disengage the ball catch and allow the detent to snap downwardly into engagement with the lock face 17 to lock the joint against flexing.

The arrangement shown in Figure 3 is generally similar to that shown in Figures 1 and 2 except that the ball 23 and ball spring 24 have been omitted. When the actuating ring 26 has been operated to retract the detent the joint may be flexed as shown in Figure 3. On release of the actuating ring the detent is supported in a partially retracted position by the arcuate peripheral edge 16 and the members 10 and 11 may be straightened. As soon as the members 10 and 11 reach the unflexed position the detent 20 automatically snaps into engagement with the lock face 17 to lock the joint against flexing.

As stated above, it is preferred to manufacture the joint as a separate sub-assembly to be fitted into the limb brace or artificial limb.

Figures 4(I) and 4(III) show two different shapes of hinged ring catch 26 fitted to the member 11, lifting the rear face causing the joint to unlock. In Figure 4(I) finger pressure is applied to the back of the actuating ring 26 and in Figure 4(III) finger pressure is applied to a rear side portion thereof. Figures 4(II) and 4(IIa) show the actuating ring 26 fitted with an extension bar 31 and arranged to operate as a bar lock. Figure 4(IV) shows the member 26 having an extension 32 whose distal end is formed with an eye for fixing the cable of a cable lift system.

It will be understood that the invention described above is not restricted to the details of the preferred forms described by way of example above.

**Claims**

1. A releasably lockable joint for an orthopaedic limb support or for an artificial limb, which joint comprises first and second members (10, 11) pivoted together at their ends for relative flexing movement and having stop faces (13, 14) which abut when the joint is straight to prevent movement beyond the straight position, the pivoted end of the first member (10) having an arcuate portion (16) which terminates in a recess or shoulder (17, 18) and the second member (11) having a channel (19) which is in register with the base of said recess or shoulder (17, 18) when the joint is straight and which extends away from the recess or shoulder (17, 18), a detent (20) slidable in the channel (19) so that it may be retracted into the channel (19) by operation of a release (26) and is supported or supportable in its retracted position by said arcuate portion (16) when the joint is flexed but is urged by resilient means (21) into engagement with said recess or shoulder (17, 18) when the joint is straightened to lock the joint in the straight position, characterised in that the release for the detent (20) is a lever (26) pivoted at one end to one longitudinal edge of the second member (11) and extending transversely of the second member (11) so that its other end projects beyond the other longitudinal edge of the second member (11) with a pivotal connection (28) to the detent (20) at an intermediate position along the length of the release lever (26).

2. A joint according to claim 1, wherein the lever is in the form of an external ring (26) fitted to the second member (11) with its front edge pivotally received in a recess (27) in the front edge of the second member (11) and the internal surface of its rear edge normally spaced from the rear edge of the second member (11), an upward movement of the rear edge of the ring (26) retracting the detent (20) into the channel (19).

3. A joint according to claim 2, wherein the rear portion of the ring (26) is provided with a socket for receiving an extension lever (31, 32) which forms part of a bar lock or cable lift device.

4. A joint according to any preceding claim, wherein a releasable catch for locking the detent (20) in the retracted position is provided, said releasable catch comprising an auxiliary channel (22) in the second member (11) opening into the detent channel (19), a ball catch (23, 24) in the auxiliary channel (22) resiliently biased to project therefrom, and a recess (25) in the detent (20) positioned to be engaged by the ball (23) of the ball catch (23, 24) when the detent (20) is retracted.

**Revendications**

1. Articulation verrouillable et déverrouillable pour un support de membre orthopédique ou pour un membre artificiel, qui comprend un premier et un second élément (10, 11) articulés l'un à l'autre à leurs extrémités de manière à se déplacer l'un par rapport à l'autre en un mouvement de flexion et comportant des faces d'arrêt (13, 14) qui viennent en contact lorsque l'articulation est redressée pour empêcher tout déplacement au-delà de la position redressée, l'extrémité pivotante du premier élément (10) comportant une partie courbe (16) qui se termine dans un évidement ou un épaulement (17, 18) et le second élément (11) comportant une rainure (19) qui coïncide avec la base de l'évidement ou de l'épaulement (17, 18) lorsque l'articulation est redressée et qui s'étend dans un sens s'éloignant de l'évidement ou de l'épaulement (17, 18), un déclic (20) pouvant coulisser dans la rainure (19, de manière à pouvoir être rétracté dans la rainure (19) par actionnement d'un moyen de déverrouillage (26) et qui est supporté ou peut être supporté dans sa position rétractée par la partie courbe (16) lorsque l'articulation est fléchie, mais est sollicité par un moyen élastique (21) en contact avec l'évidement ou l'épaulement (17, 18) lorsque l'articulation est redressée pour verrouiller l'articulation dans cette position redressée, caractérisée en ce que le moyen de déverrouillage pour le déclic (20) est un levier (26) articulé à une extrémité à un bord longitudinal du second élément (11) et s'étendant transversalement au second élément (11), de sorte que son autre extrémité dépasse de l'autre bord longitudinal du second élément (11), une articulation (28) au déclic (20) étant prévue en un endroit intermédiaire le long du levier de déverrouillage 26.

2. Articulation suivant la revendication 1, caractérisée en ce que le levier a la forme d'un anneau externe (26) monté sur le second élément (11) et dont le bord antérieur est reçu à pivot dans un évidement (27) prévu dans le bord antérieur du second élément (11), la surface interne de son bord postérieur étant normalement espacée du bord postérieur du second élément (11), un déplacement vers le haut du bord postérieur de l'anneau (26) rétractant le déclic (20) dans la rainure (19).

3. Articulation suivant la revendication 2, caractérisée en ce que la partie postérieure de l'anneau (26) est pourvue d'une douille destinée à recevoir un levier de prolongement (31, 32) qui fait partie du dispositif de verrouillage à barre ou du dispositif de levage à câble.

4. Articulation suivant l'une quelconque des revendications précédentes, caractérisée en ce qu'un arrêt déverrouillable destiné à verrouiller le déclic (20) dans la position rétractée est prévu, l'arrêt déverrouillable comprenant un passage auxiliaire (22) dont le second élément (11) s'ouvrant dans la rainure (19) du déclic, un arrêt à bille (23, 24) dans le passage auxiliaire (22) sollicité élastiquement de manière à dépasser de celui-ci et un évidement (25) dans le déclic (20) placé de manière à être attaqué par la bille (23) de l'arrêt à bille (23, 24) lorsque le déclic (20) est rétracté.

**Patentansprüche**

1. Ein lösbar blockierbares Gelenk für eine orthopädische Gliedmaßenstütze oder für ein künstliches Gliedmaß, wobei dieses Gelenk erste und zweite Teile (10, 11) umfaßt, die für eine relative Biegebewegung an deren Enden zusammen drehbar verbunden sind und Anschlagflächen (13, 14) haben, die anliegen, wenn das Gelenk gestreckt ist, um einer Bewegung jenseits der gestreckten Position vorzubeugen, wobei das drehbar gelagerte Ende des ersten Gliedes (10) einen bogenförmigen Bereich (16) hat, der in einer Ausnehmung oder einer Schulter (17, 18) endet, und das zweite Glied (11) einen Kanal (19) hat, der mit der Basis der genannten Ausnehmung oder der Schulter (17, 18) fluchtet, wenn das Gelenk gestreckt ist, und von der Aussparung oder der Schulter (17, 18) ausgeht, eine in dem Kanal (19) derart verschiebbare Sperrklinke (20) umfaßt, daß sie in den Kanal (19) durch die Bewegung eines Auslösers (26) zurückgedrückt werden kann, und die gestützt wird oder abstützbar ist in ihrer eingezogenen Stellung durch den genannten bogenförmigen Bereich (16), wenn das Gelenk gekrümmt wird, die aber durch ein federndes Element (21) im Eingriff mit besagter Ausnehmung oder Schulter (17, 18) gebracht wird, um das Gelenk in der gestreckten Stellung zu verschließen, wenn das Gelenk gestreckt wird, dadurch gekennzeichnet, daß der Auslöser für die Sperrklinke (20) ein Hebel (26) ist, der an einem Ende an einer Lägskante des zweiten Gliedes (11) drehbar gelagert ist und quer vom zweiten Glied (11) absteht, so daß sein anderes Ende über die andere Längsseite des zweiten Gliedes (11) mit einer drehbaren Verbindung (28) zu der Sperrklinke (20) in einer Zwischenstellung entlang der Länge des Auslösehebels (26) hinausragt.

2. Ein Gelenk nach Anspruch 1, bei dem der Hebel als außen ringförmiges Teil (26) ausgebildet und am zweiten Glied (11) befestigt ist, mit seiner Frontecke drehbar in einer Ausnehmung (27) in der Vorderecke des zweiten Gliedes (11) aufgenommen, und wobei die nach innen zeigende Fläche seiner rückwärtigen Ecke rechtwinklig absteht von der Rückseite des zweiten Gliedes (11), und wobei eine Aufwärtsbewegung der rückwärtigen Ecke des Ringes (26) die Sperrklinke (20) in den Kanal (19) zurückdrückt.

3. Ein Gelenk nach Anspruch 2, bei dem der rückwärtige Bereich des Ringes (26) versehen ist mit einem Ansatz zur Aufnahme eines Verlängerungshebels (31, 32), der Teil eines Stabverschlusses oder eines Kabelhubmittels bildet.

4. Ein Gelenk nach einem der vorangehenden Ansprüche, bei dem eine lösbare Arretierung zum Blockieren der Sperrklinke (20) in der zurückgedrückten Stellung vorgesehen ist, wobei die genannte lösbare Arretierung einen Hilfskanal (22) in dem zweiten Glied (11) aufweist, der in den Kanal (19) für die Sperrklinke mündet, und in der Hilfsnut (22) eine Kugelmitnahme (23, 24) federnd so eingestellt ist, daß sie daraus hervorsteht, und eine Ausnehmung (25) in der Sperrklinke (20) angeordnet ist, um von der Kugel (23) der Kugelmitnahme (23, 24) eingegriffen zu werden, wenn die Sperrklinke zurückgezogen wird.

FIG.1

FIG.2

FIG.3

0016268

FIG.4